# FASCICULE DE BREVET EUROPEEN

(11) **EP 4 051 216 B1**
(45) Date de publication et mention de la délivrance du brevet: **11.02.2026**
(21) Numéro de dépôt: 20807832.9
(22) Date de dépôt: 27.10.2020
(51) Int. Cl.: A61K 8/04, A61K 8/73, A61Q 19/00

(54) **MOUSSE GÉLIFIÉE À BASE DE POLYSACCHARIDE**
GELIERTER SCHAUM AUF POLYSACCHARID-BASIS
POLYSACCHARIDE-BASED GELIFIED FOAM

(30) Priorité: 28.10.2019 FR 1912059
(43) Date de publication de la demande: 07.09.2022
(73) Titulaire: URGO RECHERCHE INNOVATION ET DEVELOPPEMENT, 21300 Chenôve (FR); CENTRE NATIONAL DE LA RECHERCHE SCIENTIFIQUE (CNRS), 75016 Paris (FR)
(72) Inventeur: ANDRIEUX, Sébastien, 67000 Strasbourg (FR); DRENCKHAN-ANDREATTA, Wiebke, 67000 Strasbourg (FR)
(74) Mandataire: Plasseraud IP
(86) Numéro de dépôt international: PCT/FR2020/051940
(87) Numéro de publication internationale: WO 2021/084200

(56) Documents cités:
- EP-A2- 0 537 999
- WO-A1-2004/073697
- WO-A1-94/00512
- CA-A1- 2 006 882
- CA-C- 2 006 882
- US-B1- 7 128 929

## Description

La présente invention a pour objet une mousse gélifiée à base de polysaccharide. Plus précisément, l'invention a pour objet un mélange permettant l'obtention d'une telle mousse gélifiée, un procédé pour la préparation d'une telle mousse gélifiée, la mousse gélifiée ainsi obtenue, et ses utilisations.

### Domaine technique

La présente invention peut trouver des applications dans le domaine biomédical, tel que, par exemple, le comblement et/ou le traitement des plaies, la libération contrôlée d'actifs, dans le domaine alimentaire et dans le domaine cosmétique.

### Technique antérieure

Les mousses de polysaccharide, tels que par exemple, les mousses d'alginate, ont été décrites pour des applications telles que les pansements, les systèmes d'administration à libération contrôlée, la culture cellulaire, les milieux de barrière pour empêcher l'adhérence des tissus et des implants biorésorbables. Pour obtenir une mousse stable à partir de polysaccharides en solution, une étape de moussage suivie d'une étape de réticulation (covalente ou ionique) sont nécessaires. La densité de réticulation peut être contrôlée très précisément. Toutefois, la toxicité des agents réticulants covalents pousse vers une réticulation ionique pour la mise en œuvre de mousses de polysaccharide dans les domaines médicaux, alimentaires ou cosmétiques.

L'étape de moussage peut être réalisée chimiquement, par exemple au moyen d'un agent de moussage, ou physiquement, par le biais d'un mixeur, ou de tout autre système de foisonnement, ou par incorporation de gaz, par exemple au moyen d'un siphon.

La gélification ionique nécessite quant à elle une réticulation ionique partielle dudit polysaccharide au moyen d'un contre-ion polyvalent solubilisé. En effet, le sel soluble (provenant du contre-ion polyvalent solubilisé) se complexe avec les groupements carboxylates du polysaccharide solubilisé pour former un gel de polysaccharide insoluble en milieu physiologique.

Pour obtenir un gel de polysaccharide homogène en volume, il convient de mélanger de manière homogène les contre-ions polyvalents au sein du polysaccharide solubilisé avant de déclencher la réticulation ionique. En effet, la cinétique d'association des contre-ions polyvalents avec le polysaccharide solubilisé (par exemple l'alginate), est très rapide (instantanée à l'échelle humaine). Il faut donc éviter la réaction de complexation ionique entre le contre-ion polyvalent (généralement un ion métallique) et le polysaccharide solubilisé (par exemple l'alginate) pendant leur mélange, et ne déclencher la gélification qu'une fois les composés mélangés de manière homogène. Pour contrôler cette réaction, deux méthodes sont communément mises en œuvre dans la littérature.

Une première méthode consiste à introduire des contre-ions polyvalents sous forme insoluble. Par exemple, des cations métalliques tels que des ions calcium peuvent être introduits sous la forme d'une poudre de carbonate de calcium, qui peut être homogénéisée au sein du polysaccharide (alginate) en solution. Le cation polyvalent peut ensuite être dissout par ajustement du pH de la solution, généralement par acidification. Dans le cas du carbonate de calcium, l'ajout d'acide entraine sa dissociation en ions calcium et ions carbonate, lesdits ions calcium divalents permettant la réticulation ionique du polysaccharide. La cinétique de gélification du polysaccharide est uniquement dictée par le contrôle de l'acidité du milieu et la cinétique de dissolution du carbonate de calcium en milieu acide.

Une seconde méthode consiste à mettre en œuvre des cations métalliques, tel que des ions calcium solubilisés, sous forme complexée. Le complexe de cation métallique peut par exemple être obtenu au moyen d'un agent chélatant tel que l'EGTA (acide egtazique) ou l'EDTA (éthylènediaminetétraacétique), l'HEDTA (acide N-(2-hydroxyéthyl) éthylènediaminetriacétique), le DTPA (l'acide diéthylène triamine penta acétique) ou le citrate de sodium. La solution de complexe d'ion calcium peut ensuite être mélangée à la solution de polysaccharide (alginate), ou bien l'alginate peut être directement dissout dans la solution de complexe d'ions métalliques. Aucune réticulation n'a lieu car les ions métalliques complexés ne sont pas disponibles. Les ions métalliques chélatés peuvent être libérés en acidifiant le milieu pour reprotoner de l'agent chélateur, et déclencher la réticulation. La dissociation des ions métalliques et de l'agent chélateur est instantanée, tout comme l'association des ions métalliques (calcium) à l'alginate pour former des liaisons ioniques qui constituent les points de réticulation du réseau hydrogel. La cinétique de gélation du polysaccharide est uniquement dictée par l'acidité du milieu et la cinétique de dissociation des ions métalliques et de l'agent chélateur.

Le document WO2004/73697 porte sur une solution d'alginate gélifiée par un cation métallique di- ou trivalent, et moussée par une réaction chimique au moyen d'un agent moussant/effervescent tel que du carbonate ou du bicarbonate de sodium, la solution comprenant par ailleurs du tétraborate de sodium pour lui conférer de la flexibilité et de l'élasticité. Le document WO94/00512 propose par exemple de préparer une mousse d'alginate par moussage mécanique à l'aide d'un mixeur. La mousse obtenue est ensuite stabilisée par l'ajout de cations di- ou trivalents, solubles ou insolubles. Lorsqu'ils sont insolubles, l'ajout d'acide permet leur dissolution et la dissociation de sels métalliques polyvalents. Ces deux documents mettent en œuvre des méthodes de moussage par mélange mécanique, rendant difficile leur utilisation pour des applications in situ

Le document CA2006882 décrit, quant à lui, une mousse qui se forme in situ en mélangeant deux solutions : l'une contenant une solution aqueuse d'alginate, un sel métallique di- ou trivalent insoluble et optionnellement un agent effervescent pour aider au moussage, l'autre contenant un acide, et optionnellement également un alginate. Lors du mélange des deux solutions, l'acidification de la solution contenant les sels métalliques insolubles induit la gélification, et éventuellement le moussage lorsqu'un agent effervescent est présent. Ce procédé requiert un mélange parfait et rapide des solutions, ce qui suppose de formuler des solutions de faible viscosité. Or, une faible viscosité initiale des solutions impose d'importantes limitations en termes de formulation et des compromis quant aux propriétés mécaniques du système final. De plus, le document propose la mise en œuvre d'un tel système au moyen d'une seringue à double piston avec tête mélangeuse, ce qui suppose soit un produit à usage unique, soit l'usage de têtes mélangeuses stériles jetables. Enfin, si la mousse a vocation à être appliquée sur la peau ou sur une plaie, la présence d'acide peut provoquer une douleur, une irritation ou une éruption cutanée.

Ainsi, il existe un besoin pour une solution pour la préparation d'une mousse de polysaccharide gélifiée, facile d'utilisation et ne nécessitant pas la présence d'un acide. Un tel système permettrait d'éviter une étape de mélange avant application in situ - ce qui simplifierait sa mise en œuvre - et de s'affranchir de l'utilisation coûteuse de matériel stérile à usage unique. De plus, si aucun acide sous forme liquide n'est ajouté au système, cela permet de limiter la dilution du polysaccharide qui impacte négativement les propriétés mécaniques de la mousse gélifiée.

De manière surprenante, la demanderesse a trouvé qu'il était possible d'obtenir une mousse de polysaccharide, et notamment d'alginate, homogène, stable dans le temps, présentant une bonne tenue mécanique et une bonne capacité d'absorption, à partir d'une solution unique de polysaccharide ne comprenant pas d'acide.

### Résumé

L'invention a ainsi pour objet, selon un premier aspect, un procédé pour la préparation d'une mousse gélifiée de polysaccharide comprenant les étapes suivantes:
a. la préparation d'un mélange comprenant:
   - au moins un polysaccharide solubilisé choisi parmi des alginates, substances pectiques, carraghénanes, et des mélanges de ceux-ci
   - au moins un solvant dudit polysaccharide, ledit solvant étant exempt d'ion divalent,
   - au moins un agent de réticulation ionique dudit polysaccharide, ledit agent de réticulation étant un ion bivalent sous forme chélatée
   - optionnellement au moins un plastifiant soluble dans ledit solvant,
   - optionnellement au moins un tensioactif, et
   - optionnellement au moins un additif,
b. le moussage et la gélification dudit mélange préparé à l'étape a. par incorporation d'un gaz modificateur de pH, le gaz modificateur de pH étant un gaz acide, de préférence choisi parmi le dioxyde de carbone, le dioxyde de souffre et/ou l'oxyde d'azote, ou un mélange de ceux-ci, et
c. optionnellement le séchage de la mousse gélifiée obtenue à l'étape b.

Selon un second autre aspect, l'invention a pour objet une mousse gélifiée de polysaccharide obtenue à partir d'un tel procédé.

Il est fait référence à l'utilisation d'une telle mousse gélifiée dans le domaine biomédical, alimentaire ou cosmétique.

Enfin, selon un troisième aspect, l'invention a pour objet une telle mousse gélifiée pour son utilisation dans le traitement des plaies, en particulier les plaies cavitaires, les ulcères veineux, les ulcères du pied diabétique, les ulcères de pression.

### Description des modes de réalisation

Le procédé de préparation d'une mousse gélifiée de polysaccharide selon l'invention met en œuvre une première étape de préparation d'un mélange comprenant:
- au moins un polysaccharide solubilisé choisi parmi des alginates, substances pectiques, carraghénanes, et des mélanges de ceux-ci
- au moins un solvant dudit polysaccharide, ledit solvant étant exempt d'ion divalent,
- au moins un agent de réticulation ionique dudit polysaccharide, ledit agent de réticulation étant un ion bivalent sous forme chélatée
- optionnellement au moins un plastifiant soluble dans ledit solvant,
- optionnellement au moins un tensioactif, et
- optionnellement au moins un additif.

### Polysaccharide

Le polysaccharide selon l'invention est un biopolymère soluble dans l'eau choisi parmi les alginates, les substances pectiques, les carraghénanes, et leurs mélanges.

Les alginates sont des sels de l'acide alginique. L'acide alginique, qui est isolé à partir d'algues, est un acide polyuronique composé de deux acides uroniques: acide D-mannuronique et d'acide L-guluronique. Le rapport de l'acide mannuronique et acide guluronique varie en fonction de facteurs tels que l'espèce d'algue, l'âge de la plante, et une partie de l'algue (par exemple, tige, feuille).

L'acide alginique est sensiblement insoluble dans l'eau. Il forme des sels hydrosolubles avec des métaux alcalins tels que le sodium, le potassium et le lithium, le magnésium ou l'ammonium mais également avec les cations d'ammonium substitués dérivés d'aminées inférieures, telles que la méthylamine, l'éthanolamine, la diéthanolamine et la triéthanolamine. Les sels sont solubles dans les milieux aqueux au-dessus de pH 4, mais sont convertis en acide alginique lorsque le pH est abaissé en dessous d'environ pH 4. Un alginate insoluble dans l'eau est formé si certains cations polyvalents, en particulier le calcium, le baryum, le strontium, le zinc, le cuivre, l'aluminium et leurs mélanges sont présents dans le milieu à des concentrations appropriées.

Les substances pectiques comprennent les pectines et les pectates. La pectine est un polysaccharide naturel qui se trouve dans les racines, les tiges, les feuilles et les fruits de diverses plantes, en particulier la peau d'agrumes tels que citrons verts, citrons, pamplemousses et les oranges. Les pectines contiennent des motifs polymères dérivés de l'acide D-galacturonique.

Le carraghénane se réfère à un groupe de galactanes sulfatés extraits d'algues rouges. Les carraghénanes sont des chaînes linéaires d'unités D-galactopyranosyle joints à alternance (1→3)α-D et (1→4) liaisons β-D-glycosidiques. Les carraghénanes peuvent, en partie, être distingués par le degré et la position de sulfatation. La plupart des unités de sucre ont un ou deux groupes sulfates estérifiés à un groupe hydroxyle sur les carbones C-2 ou C-6. Il existe trois principaux types de carraghénane, carraghénane kappa, iota carraghénane et lambda carraghénane. Les Kappa carraghénanes produisent des gels rigides solides, tandis que ceux à base de produits iota sont flasques. Les carraghénanes Lambda ne gélifient pas dans l'eau. Un iota carraghénane est préféré.

### Selon un mode préféré de réalisation, le polysaccharide mis en œuvre dans le cadre de l'invention est l'alginate.

Le mélange préparé à l'étape a. comprend généralement de 0,5% à 10% en poids, de préférence de 1% à 6% en poids, plus préférentiellement de 2% à 4% en poids de polysaccharide, de préférence d'alginate.

De préférence, les alginates présentent une masse moléculaire en poids compris entre 50 000 et 400 000 Da, mesurée selon la méthode de chromatographie d'exclusion stérique.

Des alginates de haut poids moléculaires (entre 100 000 et 400 000 Da) peuvent être utilisés seuls ou en association avec des alginates de bas poids moléculaire (inférieur à 100 000 Da).

### Solvant

Le mélange préparé à l'étape a. du procédé revendiqué met en œuvre au moins un solvant ledit solvant étant exempt d'ion divalent.

Ces solvants permettent de solubiliser tout ou partie des ingrédients du mélange.

De préférence, le solvant est inorganique, et avantageusement, le solvant est l'eau.

Le solvant permet de dissoudre ou de disperser les ingrédients du mélange préparé à l'étape a. Le solvant utilisé pour former le mélange ne doit pas contenir des ions tels que le calcium, susceptible de réticuler le polysaccharide et de former un gel au stade de l'étape a. Ainsi, le solvant est exempt d'ion divalent. De préférence encore, le solvant peut être l'eau. Lorsque le solvant mis en œuvre est l'eau, il est préférable d'utiliser de l'eau distillée ou déionisée.

En particulier, le solvant est présent en une quantité de 50% à 99% en poids, de préférence de 80% à 97% en poids, par rapport au poids total du mélange.

### Agent de réticulation ionique

Le mélange préparé à l'étape a. du procédé revendiqué met en œuvre au moins un agent de réticulation ionique du polysaccharide, lequel agent de réticulation est non disponible, c'est-à-dire dans le contexte de l'invention l'agent de réticulation est un ion bivalent sous forme chélatée.

L'agent de réticulation ionique est un cation bi- valent ou peut être un mélange de cations bivalents capables de réticuler ioniquement avec le polysaccharide.

Selon un mode préféré de réalisation, les cations bivalents appropriés comprennent, par exemple, le calcium, le baryum, le strontium, le fer bivalent, le zinc, le cuivre bivalent. Les cations préférés sont les cations de calcium.

Des agents de réticulation ioniques peuvent être mis en œuvre sous forme de sels tels que le carbonate de calcium, l'édétate disodique de calcium, l'oxalate de calcium, le phosphate dicalcique, le phosphate tricalcique, citrate de tricalcium, le carbonate de strontium, le carbonate de baryum, le carbonate cuivrique, le carbonate de zinc, oxalate et le phosphate de zinc, leurs hydrates, et des mélanges de ceux-ci. Tout sel ou combinaison de sels qui fournit le cation bivalent souhaité pour la réticulation ou un mélange de cations bivalents peut être utilisé en tant qu'agent de réticulation ionique.

L'agent de réticulation ionique mis en œuvre à l'étape a. du procédé de l'invention est « non disponible » de sorte que le mélange mis en œuvre dans l'étape a. du procédé ne puisse pas réticuler le polysaccharide avant l'incorporation d'un gaz acide modificateur de pH. On entend par agent de réticulation « non disponible » un agent de réticulation ionique qui a été neutralisé pour ne pas réagir avec le polysaccharide sans l'ajout d'un agent activateur. L'agent de réticulation peut être rendu indisponible en étant introduit dans le mélange sous forme de composé insoluble, ou sous forme de composé complexé. L'activation dudit agent de réticulation « non disponible » est par exemple opérée par ajustement du pH au moyen d'un agent modificateur de pH, le gaz modificateur de pH est un gaz acide, en particulier par acidification.

L'agent de réticulation ionique peut être rendu « non disponible » en l'introduisant dans le mélange de l'étape a. sous une forme insoluble dans le solvant (en particulier dans l'eau), mais pouvant être solubilisé, de préférence en milieu acide, de manière à libérer le cation polyvalent, de manière à assurer la réticulation du polysaccharide et à former le gel recherché. Typiquement, le cation polyvalent peut être libéré à un pH à partir de 3, en particulier entre 3 et 6,5.

En variante, l'agent de réticulation ionique peut être soluble dans le solvant (en particulier dans l'eau), mais rendu « non disponible » par complexation au moyen d'un agent chélatant. De même que dans la précédente variante, le cation bivalent peut, de préférence, être libéré dans des conditions acides. L'agent chélatant peut par exemple être choisi parmi l'EGTA (acide egtazique) ou l'EDTA (éthylènediaminetétraacétique), l'HEDTA (acide N-(2-hydroxyéthyl) éthylènediaminetriacétique), le DTPA (l'acide diéthylène triamine penta acétique)

L'agent de réticulation ionique est un ion bivalent sous forme chélatée.

Un agent de réticulation préféré, en particulier lorsque le polysaccharide est l'alginate ou choisi parmi les matières pectiques et iota carraghénane, est le calcium chélaté par l'acide egtazique.

L'agent de réticulation ionique est présent en une quantité de 0,1% à 15% en poids, de préférence de 1% à 10% en poids, plus préférentiellement de 2% à 5% en poids, par rapport au poids total du mélange.

### Plastifiant

Le mélange préparé à l'étape a. du procédé peut également mettre en œuvre au moins un plastifiant, de préférence soluble dans le solvant, en particulier dans l'eau.

Ainsi, selon un mode préféré de réalisation, le mélange préparé à l'étape a. comprend un plastifiant soluble dans l'eau.

Un plastifiant confère de la souplesse à la mousse gélifiée de polysaccharide.

Les plastifiants typiques sont des alcools polyhydriques tels que la glycérine, le sorbitol, l'éthylène glycol, le propylène glycol et le polyéthylène glycol.

De préférence, le plastifiant est non toxique et ne modifie pas la solubilité du polysaccharide.

### Tensioactif

Le mélange préparé à l'étape a. du procédé peut également comprendre au moins un tensioactif, de préférence non ionique, comme agent moussant.

Le tensioactif non ionique peut être choisi parmi
(1) des tensioactifs qui sont fluides à une température inférieure ou égale à 45 °C, choisis parmi les esters d'au moins un polyol choisi dans le groupe formé par un polyéthylèneglycol comprenant de 1 à 60 motifs d'oxyde d'éthylène, le sorbitane, le glycérol comprenant de 2 à 30 motifs d'oxyde d'éthylène, des polyglycérols comprenant de 2 à 12 motifs de glycérol, et d'au moins un acide gras comprenant au moins une chaîne alkyle en C8 -C22 linéaire ou ramifiée, saturée ou insaturée,
(2) des esters mixtes d'acide gras ou d'alcool gras, d'acide carboxylique et de glycérol,
(3) des esters d'acide gras de glucides et des éthers d'alcool gras de sucres,
(4) des tensioactifs qui sont solides à une température inférieure ou égale à 45 °C, choisis parmi des esters d'acide gras de glycérol, des esters d'acide gras de sorbitane et des esters d'acide gras de sorbitane oxyéthylénésoxyéthylénés, des éthers d'acide gras éthoxylés et des esters d'acide gras éthoxylés,
(5) des copolymères séquences d'oxyde d'éthylène (A) et d'oxyde de propylène (B), et
(6) des tensioactifs silicones.

Les tensioactifs (1) qui sont fluides à une température inférieure ou égale à 45 °C peuvent être, en particulier :
- l'isostéarate de polyéthylèneglycol de poids moléculaire 400 ;
- l'isostéarate de diglycéryle, commercialisé par la société Solvay ;
- le laurate de glycéryle comprenant 2 motifs de glycérol, commercialisé par la société Solvay ;
- l'olétate de sorbitane, commercialisé sous le nom Span 80 par la société ICI ;
- l'isostéarate de sorbitane, commercialisé sous le nom Nikkol SI 1OR par la société Nikko ; et
- le cocoate de α-butylglucoside ou le caprate de α-butylglucoside

Les esters mixtes d'acide gras ou d'alcool gras (2), d'acide carboxylique et de glycérol, qui peuvent être utilisés en tant que tensioactif non ionique ci-dessus, peuvent être choisis en particulier parmi le groupe comprenant des esters mixtes d'acide gras ou d'alcool gras avec une chaîne alkyle contenant de 8 à 22 atomes de carbone, et de α-hydroxyacide et/ou d'acide succinique, avec le glycérol. L' α-hydroxyacide peut être, par exemple, l'acide citrique, l'acide lactique, l'acide glycolique ou l'acide malique, et des mélanges de ceux-ci.

La chaîne alkyle des acides ou alcools gras à partir desquels sont dérivés les esters mixtes qui peut être utilisée dans l'émulsion de l'invention peut être linéaire ou ramifiée, et saturée ou insaturée. Elle peut être, en particulier, des chaînes stéarate, isostéarate, linoléate, oléate, béhénate, arachidonate, palmitate, myristate, laurate, caprate, isostéaryle, stéaryle, linoléyle, oléyle, béhényle, myristyle, lauryle ou capryle, et des mélanges de celles-ci.

En tant qu'exemples d'esters mixtes qui peuvent être utilisés dans l'émulsion de l'invention, il peut être mentionné l'ester mixte de glycérol et du mélange d'acide citrique, d'acide lactique, d'acide linoléique et d'acide oléique (nom CTFA : citrate/lactate/linoléate/oléate de glycéryle) commercialisé par la société Hüls sous le nom Imwitor 375 ; l'ester mixte d'acide succinique et d'alcool isostéarylique avec le glycérol (nom CTFA: succinate d'isostéaryl-diglycéryle) commercialisé par la société Hüls sous le nom Imwitor 780 K ; l'ester mixte d'acide citrique et d'acide stéarique avec le glycérol (nom CTFA: stéarate -citrate de glycéryle) commercialisé par la société Hüls sous le nom Imwitor 370 ; l'ester mixte d'acide lactique et d'acide stéarique avec le glycérol (nom CTFA : stéarate -lactate de glycéryle) commercialisé par la société Danisco sous le nom Lactodan B30 ou Rylo LA30.

Les esters d'acide gras de sucres (3), qui peuvent être utilisés en tant que tensioactif non ionique ci-dessus, peuvent de préférence être solides à une température inférieure ou égale à 45 °C et peuvent être choisis en particulier parmi le groupe comprenant des esters ou des mélanges d'esters d'acide gras en C8 - C22 et de saccharose, de maltose, de glucose ou de fructose, et des esters ou des mélanges d'esters d'acide gras en C14 -C22 et de méthylglucose.

Les acides gras en C8 - C22 ou c14 - C22 formant le motif acide gras des esters qui peuvent être utilisés dans la présente invention comprennent une chaîne alkyle linéaire saturée ou insaturée contenant, respectivement, de 8 à 22 ou de 14 à 22 atomes de carbone. Le motif acide gras des esters peut être choisi en particulier parmi des stéarates, des béhénates, des arachidonates, des palmitates, des myristates, des laurates et des caprates, et des mélanges de ceux-ci. Des stéarates sont de préférence utilisés.

En tant qu'exemples d'esters ou de mélanges d'esters d'acide gras et de saccharose, de maltose, de glucose ou de fructose, il peut être mentionné le monostéarate de saccharose, le distéarate de saccharose et le tristéarate de saccharose et des mélanges de ceux-ci, tels que les produits commercialisés par la société Croda sous le nom Crodesta F50, F70, F110 et F160 ; et des exemples d'esters ou mélanges d'esters d'acide gras et de méthylglucose qui peuvent être mentionnés sont le distéarate de méthylglucose-polyglycéryl-3, commercialisé par la société Goldschmidt sous le nom Tego-care 450. Il peut également être mentionné des monoesters de glucose ou de maltose tels que le méthyl-o-hexadécanoyl-6-D-glucoside et le o-hexadecanoyl-6-D-maltoside.

Les éthers d'alcool gras de sucres (3), qui peuvent être utilisés en tant que tensioactif non ionique ci-dessus, peuvent être solides à une température inférieure ou égale à 45 °C et peuvent être choisis en particulier dans le groupe comprenant des éthers ou des mélanges d'éthers d'alcool gras en C8 -C22 et de glucose, de maltose, de saccharose ou de fructose, et des éthers ou des mélanges d'éthers d'un alcool gras en C₁₄-C₂₂ et de méthylglucose. Ceux-ci sont en particulier des alkylpolyglucosides.

Les alcools gras en C₈-C₂₂ ou C₁₄-C₂₂ formant le motif d'acide gras des éthers qui peuvent être utilisés comprennent une chaîne alkyle linéaire saturée ou insaturée contenant, respectivement, de 8 à 22 ou de 14 à 22 atomes de carbone. Le motif d'acide gras des éthers peut être choisi en particulier parmi des motifs décyle, cétyle, béhényle, arachidyle, stéaryle, palmityle, myristyle, lauryle, capryle et hexadécanoyle, et des mélanges de ceux-ci, tel que cétéaryle.

En tant qu'exemples d'éthers d'alcool gras de glucides, il peut être mentionné des alkylpolyglucosides tels que le décylglucoside et le laurylglucoside, qui est commercialisé, par exemple, par la société Henkel sous les noms respectifs Plantaren 2000 et Plantaren 1200, cétostéaryl-glucoside facultativement sous la forme d'un mélange avec l'alcool cétostéarylique, commercialisé par exemple, sous le nom Montanov 68 par la société SEPPIC, sous le nom Tego-care CG90 par la société Goldschmidt et sous le nom Emulgade KE3302 par la société Henkel, ainsi que l'arachidyl-glucoside, par exemple sous la forme d'un mélange d'arachidyle alcool et béhényle alcool et arachidyle glucoside, commercialisé sous le nom Montanov 202 par la société SEPPIC.

Le tensioactif utilisé est plus particulièrement le monostéarate de saccharose, le distéarate de saccharose ou le tristéarate de saccharose et des mélanges de ceux-ci, le distéarate de méthylglucose-polyglycéryl-3 et des alkylpolyglucosides.

Les esters d'acide gras de glycérol (4) qui peuvent être utilisés en tant que tensioactif non ionique ci-dessus, qui sont solides à une température inférieure ou égale à 45 °C, peuvent être choisis en particulier dans le groupe comprenant des esters formés d'au moins un acide comprenant une chaîne alkyle linéaire saturée contenant de 12 à 22 atomes de carbone et de 1 à 12 motifs de glycérol.

Ces esters peuvent être choisis en particulier parmi des stéarates, des béhénates, des arachidates et des palmitates de glycérol, et des mélanges de ceux-ci.

En tant qu'exemples de tensioactifs qui peuvent être utilisés dans la présente invention, il peut être mentionné les monostéarate, distéarate, tristéarate et pentastéarate de décaglycéryle (noms CTFA : stéarate de polyglycéryl-10, distéarate de polyglycéryl-10, tristéarate de polyglycéryl-10, pentastéarate de polyglycéryl-10), tels que les produits commercialisés sous les noms respectifs Nikkol Decaglyn 1 S, 2 S, 3 S et 5 S par la société Nikko, et le monostéarate de diglycéryle (nom CTFA: stéarate de polyglycéryl-2), tel que le produit commercialisé par la société Nikko sous le nom Nikkol DGMS.

Les esters d'acide gras de sorbitane (4) qui peuvent être utilisés en tant que tensioactif non ionique ci-dessus, qui sont solides à une température inférieure ou égale à 45 °C, peuvent être choisis dans le groupe comprenant des esters d'acide gras en C16 - c22 de sorbitane et des esters d'acide gras en C16 -C22 de sorbitane oxyéthylénésoxyéthylénés. Ils sont formés d'au moins un acide gras comprenant au moins une chaîne alkyle linéaire saturée contenant, respectivement, de 16 à 22 atomes de carbone, et de sorbitol ou de sorbitol éthoxylé. Les esters oxyéthylénésoxyéthylénés comprennent généralement de 1 à 100 motifs éthylèneglycol et de préférence de 2 à 40 motifs d'oxyde d'éthylène (EO).

Ces esters peuvent être choisis en particulier parmi des stéarates, des béhénates, des arachidates, des palmitates, et des mélanges de ceux-ci.

En tant qu'exemples du tensioactif non ionique ci-dessus qui peuvent être utilisés dans la présente invention, il peut être mentionné le monostéarate de sorbitane (nom CTFA : stéarate de sorbitane), commercialisé par la société ICI sous le nom Span 60, le monopalmitate de sorbitane (nom CTFA: palmitate de sorbitane), commercialisé par la société ICI sous le nom Span 40, et le tristéarate de sorbitane 20 EO (nom CTFA: polysorbate 65), commercialisé par la société ICI sous le nom Tween 65.

Les éthers d'acide gras éthoxylés (4) qui sont solides à une température inférieure ou égale à 45 °C, qui peuvent être utilisés en tant que tensioactif non ionique ci-dessus, sont de préférence des éthers formés de 1 à 100 motifs d'oxyde d'éthylène et d'au moins une chaîne d'alcool gras contenant de 16 à 22 atomes de carbone. La chaîne grasse des éthers peut être choisie en particulier parmi des motifs béhényle, arachidyle, stéaryle et cétyle, et des mélanges de ceux-ci, tels que le cétéaryle. Des exemples d'éthers d'acide gras éthoxylés qui peuvent être mentionnés sont des éthers d'alcool béhénylique comprenant 5, 10, 20 et 30 motifs d'oxyde d'éthylène (noms CTFA: béhéneth-5, béhéneth-10, béhéneth-20, béhéneth-30), tels que les produits commercialisés sous les noms Nikkol BBS, BB10, BB20 et BB30 par la société Nikko, et l'éther d'alcool stéarylique comprenant 2 motifs d'oxyde d'éthylène (nom CTFA: stéareth-2), tel que le produit commercialisé sous le nom Brij 72 par la société ICI.

Les esters d'acide gras éthoxylés (4) qui sont solides à une température inférieure ou égale à 45 °C, qui peuvent être utilisés en tant que tensioactif non ionique ci-dessus, sont des esters formés de 1 à 100 motifs d'oxyde d'éthylène et d'au moins une chaîne d'acide gras contenant de 16 à 22 atomes de carbone. La chaîne grasse dans les esters peut être choisie en particulier parmi des motifs stéarate, béhénate, arachidate et palmitate, et des mélanges de ceux-ci. Des exemples d'esters d'acide gras éthoxylés qui peuvent être mentionnés sont l'ester d'acide stéarique comprenant 40 motifs d'oxyde d'éthylène, tel que le produit commercialisé sous le nom Myrj 52 (nom CTFA : stéarate de PEG-40) par la société ICI, ainsi que l'ester d'acide béhénique comprenant 8 motifs d'oxyde d'éthylène (nom CTFA : béhénate de PEG-8), tel que le produit commercialisé sous le nom Compritol HD5 ATO par la société Gattefosse.

Les copolymères séquencés d'oxyde d'éthylène (A) et d'oxyde de propylène (B) (5), qui peuvent être utilisés en tant que tensioactifs selon l'invention, peuvent être choisis en particulier parmi des copolymères séquencés de formule (IV) : HO(C₂H₄₀)ₓ(C₃₁₋₁₆₀)_{y}(C₂H₄₀),H dans laquelle x, y et z sont des entiers tels que x+z est dans la plage de 2 à 100 et y est dans la plage de 14 à 60, et des mélanges de ceux-ci, et plus particulièrement parmi des copolymères séquencés de formule (IV) ayant une valeur HLB dans la plage de 8,0 à 14,0.

Le tensioactif silicone (6) en tant que tensioactif non ionique ci-dessus peut de préférence être choisi parmi ceux commercialisés par la société Dow Corning sous les noms DC 5329, DC 7439-146, DC 2-5695 et Q4-3667.

Selon un mode préféré de réalisation, le tensioactif non ionique mis en œuvre dans le mélange de l'étape a. présente une valeur HLB de 8,0 à 14,0, de préférence de 9,0 à 13,5, et plus préférentiellement de 10,0 à 13,0.

Un tel tensioactif non ionique est, de préférence, choisi parmi :
- l'isostéarate ou oléate de polyéthylèneglycol (8 à 10 moles d'oxyde d'éthylène),
- l'isocétyle de polyéthylèneglycol, l'éther de béhényle ou l'éther d'isostéaryle (8 à moles d'oxyde d'éthylène),
- le monolaurate ou dilaurate de polyglycéryle comprenant 3 à 6 motifs de glycérol,
- le mono(iso)stéarate de polyglycéryle comprenant 3 à 6 motifs de glycérol,
- le monooléate de polyglycéryle comprenant 3 à 6 motifs de glycérol, et
- le dioléate de polyglycéryle comprenant 3 à 6 motifs de glycérol.

Selon un mode de réalisation préféré de la présente invention, le tensioactif non ionique ayant une valeur HLB de 8,0 à 14,0, de préférence de 9,0 à 13,5, et plus préférablement de 10,0 à 13,0, est choisi parmi des esters d'acide gras de polyglycéryle et des esters d'acide gras mono- ou poly-oxyéthylénés.

Il est préférable que l'ester d'acide gras de polyglycéryle comprenne des esters d'un acide gras et de polyglycérine contenant 70 % ou plus de polyglycérine dont le degré de polymérisation est 4 ou plus, de préférence des esters d'un acide gras et de polyglycérine contenant une quantité égale ou supérieure à 60 % de polyglycérine dont le degré de polymérisation est compris entre 4 et 11, et plus préférablement des esters d'un acide gras et de polyglycérine contenant une quantité égale ou supérieure à 30 % de polyglycérine dont le degré de polymérisation est 5.

L'ester d'acide gras de polyglycéryle peut être choisi parmi les mono, di et tri -esters d'acide saturé ou insaturé, de préférence un acide saturé, comprenant 2 à 30 atomes de carbone, de préférence 6 à 30 atomes de carbone, et plus préférablement 8 à atomes de carbone, tel que l'acide laurique, l'acide oléique, l'acide stéarique, l'acide isostéarique, l'acide caprique, l'acide caprylique, et l'acide myristique.

Il est préférable que l'ester d'acide gras de polyglycéryle soit choisi dans le groupe constitué des laurate de PG-4, laurate de PG-5, dilaurate de PG-5, oléate de PG-5, dioléate de PG-5, tricaprylate de PG-6, myristate de PG-5, trimyristate de PG-5, stéarate de PG-5, isostéarate de PG-5, trioléate de PG-5, caprylate de PG-6, et tricaprylate de PG-6.

Il est préférable que l'ester d'acide gras mono- ou poly-oxyéthyléné ait un fragment (poly)oxyalkylène dérivé de 1 à 20 oxyalkylènes, de préférence de 3 à 15 oxyalkylènes, et plus préférablement de 8 à 10 oxyalkylènes.

Le fragment oxyalkylène peut être dérivé d'alkylèneglycols tels que l'éthylèneglycol, le propylèneglycol, le butylèneglycol, le pentylèneglycol, l'hexylèneglycol, et similaire. Le fragment oxyalkylène peut contenir un nombre de moles d'oxyde d'éthylène et/ou d'oxyde de propylène compris entre 1 et 100 et de préférence entre 2 et 50. Avantageusement, les tensioactifs non ioniques ne comprennent pas de motifs oxypropylène.

L'ester d'acide gras mono- ou poly-oxyéthyléné peut être choisi parmi les mono et di -esters d'acide saturé ou insaturé, de préférence un acide saturé, comprenant de 2 à 30 atomes de carbone, de préférence de 6 à 30 atomes de carbone, et plus préférablement de 8 à 30 atomes de carbone, tels que l'acide laurique, l'acide oléique, l'acide stéarique, l'acide isostéarique, l'acide caprique, l'acide caprylique, et l'acide myristique.

Des exemples d'esters d'acide gras mono- ou poly-oxyéthylénés qui peuvent être mentionnés comprennent des esters d'acides linéaires ou ramifiés, saturés ou insaturés en C₂-C₃₀, de préférence en C₆-C₃₀ et plus préférablement en C₈-C₂₂ de polyéthylèneglycols.

Des exemples d'esters d'acide gras mono- ou poly-oxyéthylénés qui peuvent être mentionnés comprennent les adduits d'oxyde d'éthylène avec des esters d'acide laurique, d'acide myristique, d'acide palmitique, d'acide stéarique, d'acide isostéarique, d'acide oléique ou d'acide béhénique, et des mélanges de ceux-ci, en particulier ceux contenant de 8 à 30 groupes oxyéthylène, tels que le laurate de PEG-8 à PEG-30 (sous les noms CTFA: PEG-8 laurate à PEG-30 laurate) ; le myristate de PEG-8 à PEG-30 (sous les noms CTFA: PEG-8 myristate à PEG-30 myristate) ; le palmitate de PEG-8 à PEG-30 (sous les noms CTFA: PEG-8 palmitate à PEG-30 palmitate) ; le stéarate de PEG-8 à PEG-30 (sous les noms CTFA: PEG-8 stéarate à PEG-30 stéarate) ;
l'isostéarate de PEG-8 à PEG-30 (sous les noms CTFA: PEG-8 isostéarate à PEG-30 isostéarate) ; l'oléate de PEG-8 à PEG-30 (sous les noms CTFA: PEG-8 oléate à PEG-30 oléate) ; le béhénate de PEG-8 à PEG-30 (sous les noms CTFA: PEG-8 béhénate à PEG-30 béhénate) ; et des mélanges de ceux-ci.

Il est préférable que l'ester d'acide gras de polyglycol soit choisi dans le groupe constitué de l'isostéarate de PEG-8, le stéarate de PEG-8, l'isostéarate de PEG10, l'oléate de PEG10, l'éther isocétylique de PEG10, l'éther béhénylique de PEG10 ou l'éther isostéarylique de PEG10 et un mélange de ceux-ci.

Parmi les tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C6-C24) éther carboxyliques polyoxyalkylénés, les acides alkyl(C6-C24)aryl éther carboxyliques polyoxyalkylénés les acides alkyl(C6-C24) amido éther carboxyliques polyoxyalkylénés et leurs sels, en particulier ceux comportant de 2 à 50 groupements oxyde d'éthylène, et leurs mélanges.

On utilise de préférence un agent tensioactif anionique choisi parmi les alkyl(C12-C14) sulfates de sodium, de triéthanolamine ou d'ammonium, les alkyl (C12-C14)éthersulfates de sodium oxyéthylénés à 2,2 moles d'oxyde d'éthylène, le cocoyl iséthionate de sodium et l'alphaoléfine(C14-C16) sulfonate de sodium.

Parmi les tensioactifs anioniques, on préfère utiliser selon l'invention les sels d'alkylsulfates et d'alkyléthersulfates et leurs mélanges.

Le tensioactif est de préférence présent en une quantité de 0,01 % à 10% en poids, de préférence de 0,05% à 5% en poids, plus préférentiellement de 0,1% à 2% en poids, par rapport au poids total du mélange.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les agents antimousse, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques et les filtres solaires.

En particulier, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'ibuprofène, le Kétoprofène, le Flurbiprofène, le Diclofénac, l'Acéclofénac, le Kétorolac, le Méloxicam, le Piroxicam, le Ténoxicam, le Naproxène, l'indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de CentellaAsiatica, la papaïne, les silicones, les huiles essentielles de thym, de niaouli, de romarin, d'arbre à thé et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, la metformine, l'Allantoïne ;
- les agents hydratants tels que l'acide hyaluronique, l'urée, le glycérol, les acides gras, modulateurs des aquaporines, les huiles végétales, le chitosan, certains sucres dont le sorbitol, les beurres et les cires ;
- les agents dépigmentants tels que l'acide kojique (KojicAcid SL^{®} - Quimasso (Sino Lion)), l'Arbutine (Olevatin^{®} - Quimasso (Sino Lion)), le mélange de palmitoylpropyl de sodium et d'extrait de nénuphar blanc (Sepicalm^{®} - Seppic), l'undécylénoyl phénylalanine (Sepiwhite^{®} - Seppic), l'extrait de réglisse obtenue par fermentation d'Aspergillus et éthoxydiglycol (GatulineWhitening^{®} - Gattefossé), l'acide octadécènedioïque (ODA White^{®} - Sederma), l'alpha-arbutin (Alphaarbutin^{®}, SACI-CFPA (Pentapharm)), l'extrait aqueux de feuilles Arctophylos Uva Ursi (Melfade-J^{®} - SACI-CFPA (Pentapharm)), le mélange de plante complexe Gigawhite^{®} (SACI-CFPA (Alpaflor)), la diacétylboldine (Lumiskin^{®} - Sederma), l'extrait de mandarine du Japon (Melaslow^{®} - Sederma), le mélange d'extrait de citron enrichi en acide citrique et d'extrait de concombre (Uninontan^{®}U-34 - Unipex), le mélange d'extrait de Rumex occidentalis et de vitamine C (Tyrostat^{®} 11 - Unipex), des oligopeptides (Mélanostatine 5^{®} - Unipex), le dipalmitatekojique (KAD-15^{®} - Quimasso (Sino Lion)), le complexe d'origine naturelle Vegewhite^{®} de LCW, des extraits de germe de blé (Clariskin^{®} Il - Silab), l'éthyldiaminetriacétate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline^{®} (Sederma), les protéases obtenues par fermentation de Bacillus Subtilis, le produit Linked-Papain^{®} (SACI-CFPA), la papaïne (enzyme protéolytique issue du fruit de papaye);
- les actifs restructurants (par exemple resctructurants des phanères) tels que les dérivés de silice, la vitamine E, la camomille, le calcium, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;
- les filtres solaires, tels que les filtres chimiques (Oxybenzone, Sulisobenzone, Dioxybenzone, Tinosorb S^{®}, Avobenzone, p-méthoxycinnamate de 2-éthoxyéthyle, Uvinul^{®} A+, Mexoryl^{®} XL, Méthoxycinnamate ou octinoxate d'octyle, Salicylate ou octisalate d'octyle, octyl triazone ou Uvinul^{®} T 150, salicylate de méthyle, meradimate, enzacamène, MBBT ou Tinosorb^{®} M, cyanophénylcinnamate d'octyleouParsol^{®} 340, Acide para-aminobenzoïque, Ensulizole, Parsol^{®} SLX ou Polysiloxane-15 ou Benzylidènemalonatepolysiloxane, salicylate de triéthanolamine ou salicylate de trolamine, Mexoryl^{®} SX ou acide téréphtalylidènedicamphosulfonique) et les filtres minéraux (oxydes de Zinc, dioxyde de titane, kaolin, ichtyol).
- Les agents dispersants pour les sels minéraux (carbonate de calcium et autres) commercialisés par Byk (par exemple BYK-154), ou ICL Advanced Additives (sous les marques Calgon^{®} et Lopon ^{®}).

Les agents antimousse peuvent être :
- à base d'huile (huile minérale, végétale, huile blanche ou toute autre huile insoluble dans le milieu moussant). Un antimousse à base d'huile peut également contenir une cire et/ou de la silice hydrophobe pour améliorer ses performances. Les cires typiques sont l'éthylène bis stéaramide, les cires de paraffine, les cires d'ester et les cires d'alcool gras.
- sous forme de poudre. Les antimousses en poudre sont en principe des antimousses à base d'huile sur un support particulaire tel que la silice.
- à base d'eau (différents types d'huiles et de cires dispersées dans une base d'eau). Les huiles sont souvent des huiles minérales ou des huiles végétales et les cires sont des alcools gras à longue chaîne, des savons d'acide gras ou des esters.
- à base de silicone (polymères à squelette en silicium). Ceux-ci peuvent se présenter sous forme d'émulsion à base d'huile ou d'eau. Le composé de silicone consiste en une silice hydrophobe dispersée dans une huile de silicone. Des émulsifiants sont ajoutés pour que le silicone s'étale rapidement et bien dans le milieu moussant. Le composé de silicone peut également contenir des glycols de silicone et d'autres fluides à base de silicone modifiés. Le polydiméthylsiloxane est un agent antimousse largement utilisé.
- à base de copolymères de polyéthylène glycol et de polypropylène glycol. Ils se présentent sous forme d'huiles, de solutions aqueuses ou d'émulsions à base d'eau.
- à base de polyacrylates d'alkyle.

L'additif peut être présent en une quantité de 0,5% à 20% en poids, de préférence de 0,5% à 5% en poids, plus préférentiellement de 1% à 5% en poids, par rapport au poids total du mélange.

### Gélification et moussage du polysaccharide

Le procédé selon l'invention met en œuvre une deuxième étape b. de moussage et la gélification dudit mélange préparé à l'étape a. par incorporation d'un gaz modificateur de pH, ledit gaz modificateur de pH est un gaz acide.

En effet, dans le cadre de la présente invention, le gaz employé lors de l'étape b. est un gaz susceptible d'acidifier le milieu.

Parmi les gaz susceptibles d'acidifier le milieu, on peut citer à titre d'exemple le dioxyde de carbone, le dioxyde de souffre, l'oxyde d'azote, ou un mélange de ceux-ci.

Selon un mode particulier de réalisation, l'étape b. peut être mise en œuvre par incorporation d'un mélange d'un gaz acide avec un gaz inerte.

De préférence le dioxyde de carbone ou un mélange gazeux contenant du dioxyde de carbone est utilisé.

La présence de ce gaz dans les bulles de la mousse va induire le déclenchement de la gélification du système par le moussage. Le déclenchement de la gélification se fait par la dissolution du gaz présent dans les bulles dans la phase liquide de la mousse, qui, par réaction chimique, acidifie le milieu et libère les cations bivalents (soit par dissolution d'un sel métallique introduit sous forme insoluble, soit par reprotonation d'agents chélatants dudit sel) permettant la réticulation ionique du polysaccharide. De plus, puisque le moussage induit la gélification, l'invention permet un contrôle de la cinétique de formation de l'hydrogel sans avoir à ajouter d'agent d'acidification qui dilue la matrice polymère. Enfin, l'utilisation de gaz pour induire la gélification est un avantage du fait de leur stabilité chimique et de leur faible toxicité. L'utilisation du gaz permet d'initier la gélification à la surface des bulles, et donc de favoriser la formation de mousses à cellules fermées. Au contraire, la gélification obtenue par les techniques de l'art antérieur est ordinairement initiée dans le cœur de la phase liquide.

Le moussage est de préférence un moussage physique et non chimique.

Les personnes familières du domaine des mousses liquides pourront modifier la cinétique de gélification par un contrôle de la quantité de gaz acide dissous la phase liquide. Ce contrôle peut se faire par :
- la modification du ratio de gaz acide/gaz inerte dans le mélange de gaz moussant,
- l'incorporation d'un hydrocarbure perfluoré, par exemple le perfluorohexane, dans la phase gaz, qui par un équilibrage des potentiels chimiques entre les bulles limitera la dissolution du gaz acidifiant dans la phase liquide, et de fait ralentira et/ou limitera la gélification,
- la modification de la taille des bulles, dans la mesure où la pression à l'intérieur d'une bulle est d'autant plus importante que la bulle est petite. Les petites bulles favoriseront alors la dissolution du gaz acidifiant dans la phase liquide, et de fait une gélification plus rapide.

Une mousse gélifiée peut être préparée à l'étape b. par incorporation d'un gaz modificateur de pH, à l'aide de procédés micro- ou milli-fluidiques.

Un système de spray contenant un gaz acide tel que du dioxyde de carbone comme gaz propulseur peut aussi être utilisé pour une application in situ.

Un autre procédé consiste à utiliser un siphon, de type siphon de cuisine, avec des cartouches de dioxyde de carbone en tant que gaz, ou avec tous constituants susceptibles de réagir pour former un gaz acide. Par exemple, un moussage pourrait être obtenu par mélange d'une levure et d'un sucre pour former du dioxyde de carbone et de l'éthanol.

Une personne familière avec les différents procédés de moussage pourra aisément adapter les procédés de moussage plus classiques comme l'utilisation de mélangeurs mécaniques, le bullage ou le moussage par voie chimique.

### Séchage de la mousse gélifiée

Le procédé selon l'invention peut également mettre en œuvre le séchage de la mousse gélifiée obtenue à l'étape b., au moyen de toute méthode connue de l'homme du métier. Le séchage peut, par exemple, être réalisé à l'air libre ou par lyophilisation.

### Utilisation de la mousse gélifiée

La mousse gélifiée de polysaccharide obtenue à partir du procédé selon la présente invention est de préférence destinée à être appliquées sur la peau, les plaies, les phanères ou les muqueuses. Elle peut avantageusement être utilisée sur des plaies, des brûlures ou cicatrices (qu'elles soient liées à un accident, une maladie ou des suites d'une intervention chirurgicale), et trouve une application particulièrement intéressante dans les plaies cavitaires. En effet, la mousse selon l'invention peut être appliquée d'un geste simple et rapide, et va épouser le lit de la plaie.

La présente invention est illustrée plus en détail dans l'exemple non limitatif suivant.

### Exemple

### Exemple 1

On a préparé un mélange à base d'alginate et d'ion calcium complexé par de l'EGTA comme agent chélatant (volume total de 400 mL).

Pour cela, on a d'abord procédé à la complexation de l'ion calcium par l'EGTA : on a mélangé 5,88 g de CaCl₂,2H₂O (cation divalent) et 15,2 g de EGTA sont ajoutés dans 400 mL d'eau ultra pure (MilliQ). La solution est agitée à l'aide d'un agitateur magnétique.

Le pH étant alors proche de 2, le chélatant EGTA ne se dissout pas. Le pH est élevé par ajout lent de pastilles d'hydroxyde de sodium (NaOH) jusqu'à atteindre un pH de 7. La solution devient claire à un pH d'environ 4 du fait de la dissolution de l'EGTA et de son association avec les ions calcium Ca²⁺.

3,02 g d'alginate haut poids moléculaire (368 900 Da) et 9,21 g d'alginate de faible poids moléculaire (84 430 Da) sont lentement dissous dans cette solution de calcium-EGTA à une température de 50 °C à l'aide d'une agitation mécanique. Sont ajoutés ensuite 1.01 g de Saponin (de Quillaja Saponaria Molina) comme tensioactif, à faible agitation afin d'éviter un moussage intempestif lors de la dissolution du tensioactif. Une fois l'alginate bien dissout, le pH de la solution est ajusté si besoin pour être compris entre 7 et 8.

Le mélange est ensuite versé dans un siphon à crème fouettée de 1L. Une capsule de 8 g de N₂O est intégrée au système, en maintenant le siphon la tête en bas. Une autre capsule de 8 g de CO₂ est ensuite ajoutée et le système est bien mélangé avant l'activation du siphon pour procéder au moussage.

La mousse se solidifie en un temps inférieur à 5 min. Elle est homogène, stable dans le temps, présente une bonne tenue mécanique et une bonne capacité d'absorption.

### Exemple 2

On a préparé un mélange à base d'alginate et d'ion calcium complexé par de l'EGTA comme dans l'exemple 1, mais au moyen d'un système de seringues (volume total maximum de mousse : 60 mL).

Pour cela, on a préparé 12 mL d'une solution d'alginate à 0,5% en poids, 0,03 mol/L de CaCl₂,2H₂O (cation divalent) et 0,5% en poids d'EGTA.

12 mL de la solution d'alginate est introduite dans une seringue de 60 mL, reliée par un tube à une seconde seringue contenant de l'air, et une troisième seringue contenant du CO₂ (volume total de gaz : 48 mL).

La solution d'alginate est moussée en faisant passer la solution et le/les gaz d'une seringue à l'autre de manière répétée, jusqu'à ce que la mousse obtenue soit homogène.

Différentes mousses ont été obtenues en faisant varier les volumes d'air et de dioxyde de carbone :

**[Tableau 1]**

| Valg / mL | Vair/mL | VCO2/mL | VCO2/(Vair+VCO2) | Perfluorohexane |
|---|---|---|---|---|
| 12 | 48 | 0 | 0 | non |
| 12 | 28 | 20 | 0.42 | non |
| 12 | 18 | 30 | 0.63 | non |
| 12 | 0 | 48 | 100 | non |
| 12 | 0 | 48 | 100 | oui |
| 12 | 48 | 0 | 0 | oui |

Les mousses contenant du CO₂ sont homogènes, stables dans le temps, présentent une bonne résistance mécanique et une bonne capacité d'absorption. Les mousses ne contenant pas de CO₂ ne se solidifient pas et drainent rapidement. La présence de perfluorohexane permet d'améliorer la stabilité dans le temps des mousses obtenues.

## Revendications

1. Procédé pour la préparation d'une mousse gélifiée de polysaccharide comprenant les étapes suivantes:
a. la préparation d'un mélange comprenant:
- au moins un polysaccharide solubilisé choisi parmi des alginates, substances pectiques, carraghénanes, et des mélanges de ceux-ci
- au moins un solvant dudit polysaccharide, ledit solvant étant exempt d'ion divalent,
- au moins un agent de réticulation ionique dudit polysaccharide, ledit agent de réticulation étant un ion bivalent sous forme chélatée,
- optionnellement au moins un plastifiant soluble dans ledit solvant,
- optionnellement au moins un tensioactif, et
- optionnellement au moins un additif,
b. le moussage et la gélification dudit mélange préparé à l'étape a. par incorporation d'un gaz modificateur de pH, le gaz modificateur de pH étant un gaz acide, de préférence choisi parmi le dioxyde de carbone, le dioxyde de souffre et/ou l'oxyde d'azote, ou un mélange de ceux-ci, et
c. optionnellement le séchage de la mousse gélifiée obtenue à l'étape b.

2. Procédé selon la revendication 1, **caractérisé en ce que** le polysaccharide est un alginate.

3. Procédé selon la revendication 1 ou 2, **caractérisé en ce que** le polysaccharide, de préférence l'alginate, est présent en une quantité de 0,5% à 10% en poids, de préférence de 1% à 6% en poids, plus préférentiellement de 2% à 4% en poids, par rapport au poids total du mélange.

4. Procédé selon l'une quelconque des revendications précédentes, **caractérisé en ce que** l'agent de réticulation ionique est au moins un cation polyvalent, de préférence biou trivalent, et en particulier un cation bivalent.

5. Procédé selon l'une quelconque des revendications précédentes, **caractérisé en ce que** l'agent de réticulation ionique est présent en une quantité de 0,1 % à 15% en poids, de préférence de 1% à 10% en poids, plus préférentiellement de 2% à 5% en poids, par rapport au poids total du mélange.

6. Procédé selon l'une quelconque des revendications précédentes, **caractérisé en ce que** le solvant est un solvant inorganique, et de préférence le solvant est l'eau.

7. Procédé selon l'une quelconque des revendications précédentes, **caractérisé en ce que** le solvant est présent en une quantité de 50% à 99% en poids, de préférence de 80% à 97% en poids, par rapport au poids total du mélange.

8. Procédé selon l'une quelconque des revendications précédentes, **caractérisé en ce que** le tensioactif est un tensioactif non ionique ou anionique.

9. Procédé selon l'une quelconque des revendications précédentes, **caractérisé en ce que** le tensioactif est présent en une quantité de 0,01% à 10% en poids, de préférence de 0,05% à 5% en poids, plus préférentiellement de 0,1% à 2% en poids, par rapport au poids total du mélange.

10. Procédé selon l'une quelconque des revendications précédentes, **caractérisé en ce que** l'additif choisi parmi les actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques.

11. Procédé selon l'une quelconque des revendications précédentes, **caractérisé en ce que** l'additif est présent en une quantité de 0,5% à 20% en poids, de préférence de 0,5% à 5% en poids, plus préférentiellement de 1% à 5% en poids, par rapport au poids total du mélange.

12. Procédé selon l'une quelconque des revendications précédentes, **caractérisé en ce que** l'étape b. est mise en œuvre par incorporation d'un mélange dudit gaz acide avec un gaz inerte.

13. Mousse gélifiée de polysaccharide obtenue à partir du procédé selon l'une quelconque des revendications précédentes.

14. Mousse gélifiée de polysaccharide selon la revendication 13 pour son utilisation dans le traitement des plaies, en particulier les plaies cavitaires, les ulcères veineux, les ulcères du pied diabétique, les ulcères de pression.

## Patentansprüche

1. Verfahren zur Herstellung eines gelierten Polysaccharidschaums, umfassend die folgenden Schritte:
a. Herstellen einer Mischung, umfassend:
- wenigstens ein löslich gemachtes Polysaccharid, gewählt aus Alginaten, Pektinstoffen, Carrageenen und Mischungen davon,
- wenigstens ein Lösungsmittel für das Polysaccharid, wobei das Lösungsmittel frei von zweiwertigen Ionen ist,
- wenigstens ein ionisches Vernetzungsmittel für das Polysaccharid, wobei das Vernetzungsmittel ein zweiwertiges Ion in chelatisierter Form ist,
- optional wenigstens einen in dem Lösungsmittel löslichen Weichmacher,
- optional wenigstens ein Tensid, und
- optional wenigstens ein Additiv,
b. Schäumen und Gelieren der in Schritt a. hergestellten Mischung durch Einbringen eines pH-modifizierenden Gases, wobei das pH-modifizierende Gas ein saures Gas ist, bevorzugt gewählt aus Kohlendioxid, Schwefeldioxid und/oder Stickstoffoxid oder einer Mischung davon, und
c. optional Trocknen des in Schritt b. erhaltenen gelierten Schaums.

2. Verfahren nach Anspruch 1, **dadurch gekennzeichnet, dass** das Polysaccharid ein Alginat ist.

3. Verfahren nach Anspruch 1 oder 2, **dadurch gekennzeichnet, dass** das Polysaccharid, bevorzugt das Alginat, in einer Menge von 0,5 bis 10 Gew.-%, bevorzugt von 1 bis 6 Gew.-%, stärker bevorzugt von 2 bis 4 Gew.-%, bezogen auf das Gesamtgewicht der Mischung, vorliegt.

4. Verfahren nach einem der vorhergehenden Ansprüche, **dadurch gekennzeichnet, dass** das ionische Vernetzungsmittel wenigstens ein mehrwertiges, bevorzugt zweioder dreiwertiges Kation und insbesondere ein zweiwertiges Kation ist.

5. Verfahren nach einem der vorhergehenden Ansprüche, **dadurch gekennzeichnet, dass** das ionische Vernetzungsmittel in einer Menge von 0,1 bis 15 Gew.-%, bevorzugt 1 bis 10 Gew.-%, stärker bevorzugt 2 bis 5 Gew.-%, bezogen auf das Gesamtgewicht der Mischung, vorhanden ist.

6. Verfahren nach einem der vorhergehenden Ansprüche, **dadurch gekennzeichnet, dass** das Lösungsmittel ein anorganisches Lösungsmittel ist und bevorzugt Wasser ist.

7. Verfahren nach einem der vorhergehenden Ansprüche, **dadurch gekennzeichnet, dass** das Lösungsmittel in einer Menge von 50 bis 99 Gew.-%, bevorzugt von 80 bis 97 Gew.-%, bezogen auf das Gesamtgewicht der Mischung, vorliegt.

8. Verfahren nach einem der vorhergehenden Ansprüche, **dadurch gekennzeichnet, dass** das Tensid ein nichtionisches oder anionisches Tensid ist.

9. Verfahren nach einem der vorhergehenden Ansprüche, **dadurch gekennzeichnet, dass** das Tensid in einer Menge von 0,01 bis 10 Gew.-%, bevorzugt von 0,05 bis 5 Gew.-%, stärker bevorzugt von 0,1 bis 2 Gew.-%, bezogen auf das Gesamtgewicht der Mischung, vorliegt.

10. Verfahren nach einem der vorhergehenden Ansprüche, **dadurch gekennzeichnet, dass** der Zusatzstoff aus den Wirkstoffen gewählt ist, insbesondere aus antibakteriellen Mitteln, Antiseptika, antiviralen Mitteln, Antimykotika, Schmerzmitteln, Entzündungshemmern, die Wundheilung fördernden Mitteln, Feuchtigkeitsmitteln, Depigmentierungsmitteln, keratolytischen Mitteln, restrukturierenden Wirkstoffen, Anästhetika.

11. Verfahren nach einem der vorhergehenden Ansprüche, **dadurch gekennzeichnet, dass** der Zusatzstoff in einer Menge von 0,5 bis 20 Gew.-%, bevorzugt von 0,5 bis 5 Gew.-%, stärker bevorzugter von 1 bis 5 Gew.-%, bezogen auf das Gesamtgewicht der Mischung, vorhanden ist.

12. Verfahren nach einem der vorhergehenden Ansprüche, **dadurch gekennzeichnet, dass** Schritt b. durch Einbringen einer Mischung des sauren Gases mit einem Inertgas durchgeführt wird.

13. Gelierter Polysaccharidschaum, erhalten nach einem der vorhergehenden Verfahren.

14. Gelierter Polysaccharidschaum nach Anspruch 13 zur Verwendung bei der Behandlung von Wunden, insbesondere Hohlraumwunden, venösen Geschwüren, diabetischen Fußgeschwüren und Druckgeschwüren.

## Claims

1. Method for preparing a polysaccharide gelled foam comprising the following steps:
a. preparing a mixture comprising:
- at least one dissolved polysaccharide chosen from alginates, pectic substances, carrageenans, and mixtures thereof
- at least one solvent of said polysaccharide, said solvent having no divalent ions,
- at least one ionic crosslinking agent for said polysaccharide, said crosslinking agent being a bivalent ion in chelated form,
- optionally at least one plasticizer soluble in said solvent,
- optionally at least one surfactant, and
- optionally at least one additive,
b. foaming and gelling said mixture prepared in step a. by incorporating a pH-modifying gas, the pH modifying-gas being an acid gas, preferably chosen from carbon dioxide, sulphur dioxide and/or nitrogen oxide, or a mixture thereof, and
c. optionally drying the gelled foam obtained in step b.

2. Method according to claim 1, **characterised in that** the polysaccharide is an alginate.

3. Method according to claim 1 or 2, **characterised in that** the polysaccharide, preferably alginate, is present in an amount of 0.5% to 10% by weight, preferably 1% to 6% by weight, more preferably 2% to 4% by weight, relative to the total weight of the mixture.

4. Method according to any one of the preceding claims, **characterised in that** the ionic crosslinking agent is at least one polyvalent cation, preferably bivalent or trivalent, and in particular a bivalent cation.

5. Method according to any one of the preceding claims, **characterised in that** the ionic crosslinking agent is present in an amount of 0.1% to 15% by weight, preferably 1% to 10% by weight, more preferably 2% to 5% by weight, relative to the total weight of the mixture.

6. Method according to any one of the preceding claims, **characterised in that** the solvent is an inorganic solvent, and preferably the solvent is water.

7. Method according to any one of the preceding claims, **characterised in that** the solvent is present in an amount of 50% to 99% by weight, preferably 80% to 97% by weight, relative to the total weight of the mixture.

8. Method according to any one of the preceding claims, **characterised in that** the surfactant is a nonionic or anionic surfactant.

9. Method according to any one of the preceding claims, **characterised in that** the surfactant is present in an amount of 0.01% to 10% by weight, preferably from 0.05% to 5% by weight, more preferably from 0.1% to 2% by weight, relative to the total weight of the mixture.

10. Method according to any one of the preceding claims, **characterised in that** the additive is chosen from the active ingredients, in particular chosen from antibacterial agents, antiseptics, antivirals, antifungal agents, painkillers, anti-inflammatory agents, wound-healing promoters, moisturising agents, depigmenting agents, keratolytic agents, restructuring active ingredients and anaesthetics.

11. Method according to any one of the preceding claims, **characterised in that** the additive is present in an amount of 0.5% to 20% by weight, preferably 0.5% to 5% by weight, more preferably 1% to 5% by weight, relative to the total weight of the mixture.

12. Method according to any one of the preceding claims, **characterised in that** step b. is implemented by incorporating a mixture of said acid gas with an inert gas.

13. Polysaccharide gelled foam obtained from the method according to any one of the preceding claims.

14. Polysaccharide gelled foam according to claim 13 for use in the treatment of wounds, in particular cavity wounds, venous ulcers, diabetic foot ulcers and pressure ulcers.
